# EUROPEAN PATENT APPLICATION

(11) **EP 3 812 459 A1**
(43) Date of publication of application: **28.04.2021**
(21) Application number: 19823744.8
(22) Date of filing: 13.03.2019
(51) Int. Cl.: C12N 5/10, C12N 15/85, A61P 35/00

(54) **UNIVERSAL CAR-T CELL AND PREPARATION METHOD AND USE THEREOF**

(30) Priority: 20.06.2018 CN 201810636386
(71) Applicant: Shanghai Longyao Biotechnology Inc., Ltd, Shanghai 200072 (CN)
(72) Inventor: YANG, Xuanming, Shanghai 200072 (CN); FU, Yangxin, Shanghai 200072 (CN); WANG, Xin, Shanghai 200072 (CN); YE, Shengqin, Shanghai 200072 (CN); ZHANG, Xiaoqing, Shanghai 200072 (CN)
(74) Representative: Böhm, Brigitte
(86) International application number: PCT/CN2019/077921
(87) International publication number: WO 2019/242337

(57) **Abstract**

Disclosed are a universal CAR-T cell knocking out one or more of CD3 delta, CD3 gamma, CD3 epsilon and CD3 zeta, and simultaneously introducing the HSV-TK gene. Also disclosed are a method for preparing the above-mentioned CAR-T cell, a preparation comprising the CAR-T cell, and the use of the CAR-T cell.

## Description

### Technical Field

The present invention relates to the technical field of cell immunotherapy, especially relates to a universal CAR-T cell and a preparation method thereof and use thereof.

### Background

The use of immunological therapy for overcoming tumors has always been an important direction in the application of immunology in translational medicine. With the development of various omics (genomics, proteomics, etc.), tumor cells have been widely recognized due to their immunogenicity caused by mutations, which lays a theoretical foundation for tumor immunotherapy. At the same time, with the accumulation of tumor immunology research itself, tumor immunotherapy has recently made a great progress, and a series of new immunotherapy methods have gradually entered into the clinic. The current tumor immunology research has established the central position of T cell killing in tumor immunotherapy, and the chimeric antigen receptor T cell (CAR-T cell) is a tumor-killing cell which has combined the targeted recognition of antibody and the tumor-killing function of T cell, and been generated by artificial modification.

The concept of chimeric antigen receptor T cell was first proposed by Gross, Waks and Eshhar in 1989. They expressed TNP-recognizing antibodies on T cells, achieving antigen-specific, non-MHC-restricted T cell activation and enhanced effect, and proposed the concept of the application of CAR-T technology in tumor treatment. According to this principle, tumor-specific antibodies are embedded into T cells, which will give T cells new tumor-killing capabilities. After that, CAR-T technology was introduced into anti-tumor clinical trials, but the final clinical results of early CAR-T cells are not ideal since their intracellular signal transmission domain contains only the first signal, and the selected tumor type is a solid tumor. In 2008, the Fred Hutchison Cancer Institute and other institutions used CAR-T to treat B cell lymphoma, although the treatment results are not ideal, the key to this clinical trial is to demonstrate that CAR-T treatment with CD20-expressing B cells as the target is relatively safe. Subsequently, in 2010, NCI reported a case of successful treatment of B-cell lymphoma, using CAR-T targeting CD19, the patient's lymphoma was controlled, normal B cells were also eliminated, and serum Ig was significantly reduced, providing a theoretical and practical support for the effectiveness of CAR-T in the treatment of B cell-derived lymphomas. In 2011, a team led by Dr. Carl June of the University of Pennsylvania in the United States used CAR-T that specifically recognizes CD19 for the treatment of chronic lymphocytic leukemia derived from B cells, showing a "cure" effect. After that, clinical trials have been launched in relapsed and refractory acute lymphoblastic cell leukemia, and good results have also been achieved. Due to this breakthrough progress and the development of other immune regulation methods, Science magazine ranked tumor immunotherapy as the number one scientific and technological breakthrough in 2013. This success has caused widespread influence in countries around the world, and countries have begun to carry out a large number of CAR-T-based scientific research and clinical trials of tumor treatment.

The structure of CAR consists of an extracellular antigen recognition domain, an extracellular hinge region, a transmembrane domain, and an intracellular signal transduction domain. The extracellular antigen recognition domain generally consists of a single-chain antibody, which specifically recognizes membrane surface molecules of the tumor cell, or can be a ligand or a receptor of some tumor-specific antigens, etc. The extracellular hinge region is a spatial structure that separates the antigen recognition domain from the transmembrane domain, and its purpose is to provide a suitable spatial position, so that the extracellular antigen recognition domain can maintain the correct structure and transmit the intracellular signals before and after recognizing the antigen. The transmembrane domain is a domain for ensuring the positioning of the CAR molecule on the membrane surface. The intracellular signal transduction domain is a key part of mediating the CAR signal transduction, and is usually a combination of one or several first signals (for the recognition of TCR and MHC-I-peptide complex) and second signals (for the recognition of costimulatory receptor and costimulatory ligand). The first-generation CAR contains only the first signal, the second-generation CAR has one first signal and one second signal, and the third-generation CAR has one first signal and two second signal domains. Although CAR-T has achieved a great success in the treatment of leukemia derived from B cell, its relatively high recurrence rate and low effectiveness for solid tumors are important challenges currently. Therefore, there is currently an urgent clinic need of developing a new generation of high-efficiency CAR-T. In addition to the third-generation CAR-T, there are currently other new CAR-T design strategies, that is, introducing new regulatory molecules independent of CAR on the basis of the second-generation CAR-T to further enhance the function of CAR-T.

The application of CAR-T targeting the B cell surface-targeting molecules CD19 and CD20 prepared from the patient's own blood cells in the treatment of B cell leukemia has been relatively mature, but the entire process is complicated and time-consuming, while the autoimmune cells are not convenient to use as a source of T-cells for CAR-T for some special patients, such as those with serious conditions, poor quality of cells, or AIDS associated lymphoma. Although CAR-T has achieved a great success in the treatment of leukemia derived from B cells, the entire CAR-T treatment is time-consuming and has patient heterogeneity. Some patients cannot effectively produce CAR-T cells due to their own cell defects. These limit the application range of CAR-T. The development of universal CAR-T will largely solve these challenges.

Currently, all the clinic universal CAR-T protocols use CRISPR/Cas9 or TALEN gene editing means to knock out TCR so as to avoid the GVHD effect. Any other preparation method of universal CAR-T that can effectively avoid the GVHD effect and the combination with a CAR-T close switch have yet not been reported.

### Summary of the Invention

The present invention aims to address the defects in the prior art, provides an universal CAR-T cell, which achieves an effect of avoiding GVHD effect by knocking out CD3Delta, CD3Gamma, CD3 Epsilon and CD3 zeta, and introduces the HSV-TK gene. When a side effect occurs, it can be treated by the clinically existing ganciclovir to remove the CAR-T cells.

To achieve the aforesaid object, the present invention uses the following technical solutions:
a first object of the present invention is to provide a universal CAR-T cell in which one or more of CD3Delta, CD3Gamma, CD3 Epsilon and CD3 zeta is knocked out.

For further optimizing the aforesaid CAR-T cell, the technical means taken by the present invention further includes:
Further, the HSV-TK gene is introduced into the CAR-T cell.

Further, the intracellular signal transduction domain further includes at least one of CD3zeta, CD28, CD137, 4-1BB, ICOS, OX40, IL-12, 41BB, CD28, IL7R, IL2R.

Further, the extracellular antigen recognition domain is a single chain antibody or a ligand or receptor of a tumor-specific antigen, wherein the single chain antibody includes anti-CD19 antibody, anti-CD20 antibody, EGFR antibody, HER2 antibody, EGFRVIII antibody, and the ligand or receptor of tumor-specific antigen includes NKG2D;

Further, the extracellular hinge region is a region selected from CD8a or IgG; and the transmembrane domain is one selected from CD8a, CD28, CD137 or CD3.

A second object of the present invention is to provide a method of preparing a universal CAR-T cell, including the following steps: one or more of CD3Delta, CD3Gamma, CD3 Epsilon and CD3 zeta are knocked out in the CAR-T cell by using a suitable gene knockout method.

For further optimizing the aforesaid method of preparing the universal CAR-T cell, the technical means as used in the present invention further include:
Further, the preparation method further includes the following steps: performing HSV-TK gene modification in the T cell, wherein the step is implemented by conventional technical means in the art.

Further, the gene knockout method is any suitable method in the art, more preferably a CRISPR/Cas9 knockout method.

Further, the universal CAR-T cell is prepared by a gene knockout method comprising the following steps:
step 1: construction of lentiviral vector and production of virus;
   Designing an sgRNA for one or more of CD3Delta, CD3Gamma, CD3 Epsilon and CD3 zeta, cloning it into pLenti-CrisprV2, and co-transfecting it with a lentiviral packaging plasmid; after a predetermined period of time, collecting a supernatant, filtering it and performing centrifugation to concentrate the virus, thereby obtaining a plenti-CRISPRV2-sgRNA virus;
step 2: preparation of CD3-negative CAR-T cell;

Human PBMC are isolated and purified, and then inoculated into a culture plate with suitable stimulation conditions; after being cultured for a predetermined period of time, the cells are transfected with a CAR virus and the plenti-CRISPRV2-sgRNA virus produced in Step 1, and subjected to cell expansion with suitable stimulation conditions; and the CD3-positive cells are removed from the obtained cells to get the CD3-negative CAR-T cells.

Further, the specific steps of Step 1 comprise: designing an sgRNA for CD3Delta, CD3Gamma, CD3 Epsilon, CD3 zeta by using crispr.mit.edu, and cloning it into pLenti-CrisprV2; subjecting the clones which are sequenced correctly to a large-scale extraction, and co-transfecting them with lentiviral packaging plasmid into 293X; after 48 and 72 hours, collecting the supernatant, filtering it with a 0.45 uM filter, and performing centrifugation at 25000RPM for 2 hours to concentrate the virus, thereby obtaining the plenti-CRISPRV2-sgRNA virus.

Further, the stimulation conditions of culturing the isolated and purified human PBMC include anti-hCD3 and anti-hCD28, and the stimulation conditions of expanding the cells include stimulus with artificial antigen-presenting cells or anti-hCD3/28 every 6 days.

Further, the specific steps of Step 2 comprise: purifying the human PBMC by a Stemcell T cell isolation kit (negative selection), inoculating it into a 96-well plate coated with anti-hCD3 and anti-hCD28. After 2 days, infecting the cells with the CAR virus and the plenti-CRISPRV2-sgRNA virus obtained in Step 1 at MOI = 10-20. After 1 day, continuing to culture the cells with medium changed, and stimulated with artificial antigen-presenting cells or anti-hCD3/28 every 6 days. The obtained cells are treated by the Stemcell T cell positive-selection kit to remove the CD3-positive cells, thereby geting the CD3-negative 20BBZ CAR-T cells.

Further, the lentiviral packaging plasmid used in Step 1 includes VSV-g, pMD Gag/Pol, RSV-REV. The centrifugation is performed by using Beckman ultracentrifuge and SW28 head.

Further, the human PBMC is mononuclear cells derived from cord blood or adult peripheral blood.

A third object of the present invention is to provide a formulation including the aforesaid CAR-T cells or the CAR-T cells prepared by the aforesaid method. Further, the formulation also includes a pharmaceutically acceptable diluent or excipient.

A fourth object of the present invention is to provide a use of the aforesaid CAR-T cells or the CAR-T cells prepared by the aforesaid method in preparation of a medicament for treating or preventing tumor.

Further, the tumor include solid tumor and nonsolid tumor, wherein the solid tumor includes, but is not limited to, lymphomas, renal tumors, neuroblastoma, germ cell tumor, osteosarcoma, chondrosarcoma, soft tissue sarcoma, liver tumor, thymoma, pulmonary blastoma, pancreatoblastoma, hemangioma, etc.

As compared with the prior art, the present invention has the following beneficial effects:
The present invention utilizes PBMC derived from cord blood or peripheral blood, and first utilizes CRISPR/Cas9 to knock out CD3 (CD3Delta, CD3Gamma, CD3 Epsilon, CD3 zeta) to achieve an effect of avoiding GVHD, thereby constructing a universal CAR-T cell, improving the ease of use and scope of application of CAR-T cell therapy. At the same time, the present invention introduces the HSV-TK gene, thereby further improving the safety of universal CAR-T cell.

The universal CAR-T cell of the present invention exhibits a low graft-versus-host response (GVHD), and greatly enhances and expands the convenience of CAR-T cell therapy.

### Brief Description of the Drawings

FIG. 1 is a schematic structural view of the 20BBZ CAR molecule used in an embodiment of the present invention;
FIG. 2 is a schematic view of the results of the phenotypic analysis of the CD3-negative 20BBZ CAR-T cells in an embodiment of the present invention;
FIG. 3 is a schematic view showing the regulation of ganciclovir on the survival of the CD3-negative 20BBZ CAR-T cells in an embodiment of the present invention;
FIG. 4 is a schematic view of the tumor-killing ability of the CD3-negative 20BBZ CAR-T cells and the control CAR-T cells in an embodiment of the present invention;
FIG. 5 is a schematic view of the *in vivo* survival ability of the CD3-negative 20BBZ CAR-T cells and the control CAR-T cell in an embodiment of the present invention.

### Detailed Description of the Invention

A universal CAR-T cell in which CD3Delta, CD3Gamma, CD3 Epsilon and CD3 zeta are knocked out, and an HSV-TK gene is introduced is provided. The present invention also relates to a method of preparing the aforesaid CAR-T cell, a formulation including the CAR-T cell and a use of the CAR-T cell.

Hereinafter the embodiments of the present invention are further described with reference to the accompanying drawings and examples. The following examples are only for more clearly illustrating the technical solutions of the present invention, but not for limiting the protective scope of the present invention.

### EXAMPLE 1 - Preparation of CD3-Negative 20BBZ CAR-T Cells

The preparation of the CD3-negative 20BBZ CAR-T cell of this example includes the following steps:
1. Construction of Lentiviral Vector pLenti-CrisprV2-sgRNA and Production of Virus
   Designing an sgRNA for CD3Delta, CD3Gamma, CD3 Epsilon, CD3 zeta by using crispr.mit.edu, and cloning it into pLenti-CrisprV2. Subjecting the clones sequenced correctly to a large scale endotoxin-free extraction, and co-transfecting them with a lentiviral packaging plasmid (VSV-g, pMD Gag/Pol, RSV-REV) into 293X. After 48 and 72 hours, collecting a supernatant, filtering it with a 0.45 uM filter, and performing centrifugation with Beckman ultracentrifuge and SW28 head at 25000RPM for 2 hours to concentrate the virus to obtain plenti-CRISPRV2-sgRNA virus, which was used for the subsequent production of CAR-T cells.
2. Preparation of CD3-Negative 20BBZ CAR-T Cells

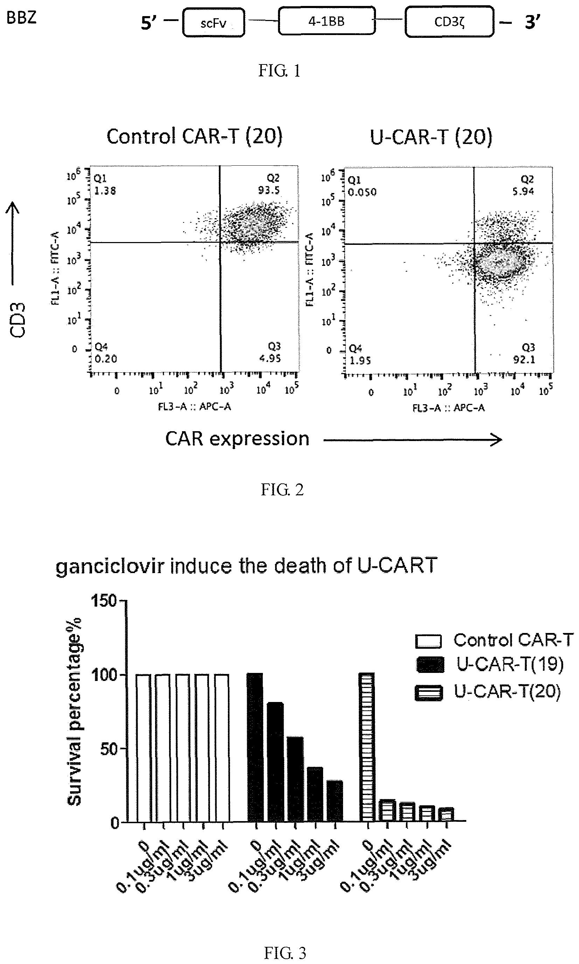

Purifying human PBMC by a Stemcell T cell isolation kit (negative selection), and then inoculating it into a 96-well plate coated with anti-hCD3 and anti-hCD28. After 2 days, infecting the cells with 20BBZ (its structure is shown in FIG. 1, and the antibody extracellular antigen recognition domain used therein is anti-CD20 antibody) and plenti-CRISPRV2-sgRNA virus at MOI= 10-20. After 1 day, continuing to culture the cells with the medium changed, and stimulating them with the artificial antigen-presenting cell or anti-hCD3/28 every 6 days. Removing the CD3-positive cells from the obtained cells by Stemcell T cell positive selection kit , thereby getting the CD3-negative 20BBZ CAR-T cells (CD3- U-CAR-T, briefly, U-CAR-T cell), which were used for the subsequent experiments and the phenotypic analysis, and the results are shown in FIG. 2. It can be seen from the figure that the obtained U-CAR-T cells are CAR-positive and CD3-negative.

### EXAMPLE 2 - The survival of U-CAR-T cell is regulated by ganciclovir

The U-CAR-T cells obtained in Step 2 of EXAMPLE 1 and the control CAR-T cells were inoculated into 96-well plates, and ganciclovir with a concentration as shown was added. After 48 hours, the CAR-T cells were compared with the survival numbers, and the results are shown in FIG. 3. It can be seen from the figure that ganciclovir can regulate the survival of U-CAR-T, and can rapidly clear the U-CAR-T from the body in the case that the U-CAR-T causes a side effect, thereby improving the safety.

### EXAMPLE 3- Comparison of Tumor-Killing Ability of U-CAR-T and Control CAR-T

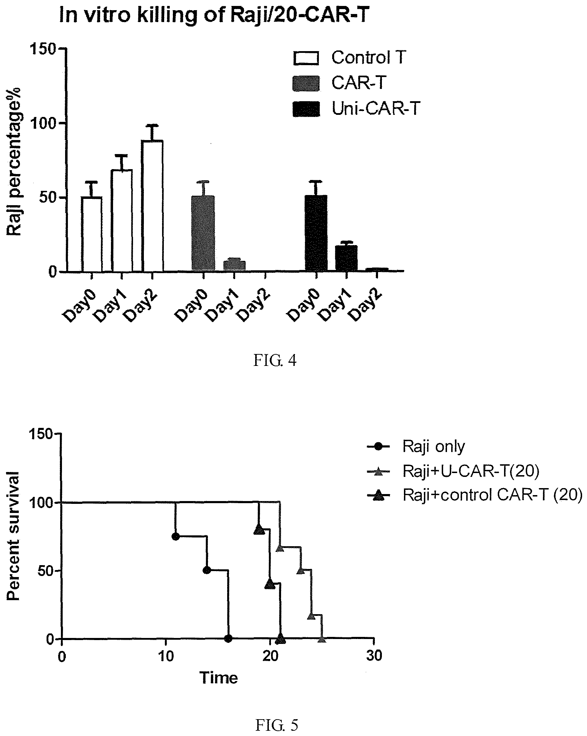

The U-CAR-T cells obtained in Step 2 of EXAMPLE 1 and the control CAR-T cells were inoculated into 96-well plates, and Raji tumor cells were added at a CAR-T: tumor cells ratio of 1:1. After 24 and 48 hours, the survival rates of the tumor cells were compared, and the results are shown in FIG. 4. It can be seen from the figure that the U-CAR-T has a similar tumor killing ability to that of the control CAR-T.

### EXAMPLE 4 - Comparison of In Vivo Survival Ability of U-CAR-T and Control CAR-T

10⁶ Raji tumor cells were intravenously inoculated into B-NDG mice. After 6 days, the mice were treated with 10⁷ U-CAR-T and the control CAR-T, and observed for their survival rate. The results are shown in FIG 5. It can be seen from the figure that both the U-CAR-T and the control CAR-T result in the prolonged survival time of the mice.

It can be seen from the aforesaid examples that, the universal CAR-T constructed by knockout of CD3 in the present invention exhibits a low graft-versus-host response (GVHD), and greatly enhances and expands the convenience of CAR-T cell therapy. Meanwhile, an HSV-TK is introduced, so that the U-CAR-T can be rapidly cleared from the body by the regulation of ganciclovir, thereby further improving the safety of the universal CAR-T.

Hereinbefore the specific embodiments of the present invention are described in details. However, they are only used as examples, and the present invention is not limited to the specific embodiments as described above. For those skilled in the art, any equivalent modifications and substitutions made to the present invention are encompassed in the scope of the present invention. Therefore, all the equal transformations and modifications without departing from the spirit and scope of the present invention should be covered in the scope of the present invention.

## Claims

1. A universal CAR-T cell, wherein one or more of CD3Delta, CD3Gamma, CD3 Epsilon and CD3 zeta is knocked out in said CAR-T cell.

2. The universal CAR-T cell according to claim 1, wherein an HSV-TK gene is introduced into said CAR-T cell.

3. A method of preparing said universal CAR-T cell according to claim 1 or 2, comprising the following steps: one or more of CD3Delta, CD3Gamma, CD3 Epsilon and CD3 zeta is knocked out in the CAR-T cell by a suitable gene knockout method.

4. The method of preparing said universal CAR-T cell according to claim 3, further comprising the following step: performing HSV-TK gene modification in a T cell.

5. The method of preparing said universal CAR-T cell according to claim 3, wherein said universal CAR-T cell is prepared by a gene knockout method comprising the following steps:
step 1: construction of lentiviral vector and production of virus;
designing an sgRNA for one or more of CD3Delta, CD3Gamma, CD3 Epsilon and CD3 zeta, cloning said sgRNA into pLenti-CrisprV2, and co-transfecting it with a lentiviral packaging plasmid; after a predetermined period of time, collecting a supernatant, filtering it and performing centrifugation to concentrate the virus, thereby obtaining a plenti-CRISPRV2-sgRNA virus;
step 2: preparation of CD3-negative CAR-T cell;
human PBMC is isolated and purified, and then inoculated into a culture plate with suitable stimulation conditions; after being cultured for a predetermined period of time, the cells are transfected with a CAR virus and the plenti-CRISPRV2-sgRNA virus produced in Step 1, and subjected to cell expansion with suitable stimulation conditions; and CD3-positive cells are removed from the obtained cells to get the CD3-negative CAR-T cells.

6. The method of preparing said universal CAR-T cell according to claim 5, wherein the stimulation conditions for culturing the isolated and purified human PBMC are anti-hCD3 and anti-hCD28, and the stimulation conditions for expanding the cells are stimulating with artificial antigen-presenting cells or anti-hCD3/28 every 6 days.

7. The method of preparing said universal CAR-T cell according to claim 5, wherein the lentiviral packaging plasmid in said Step 1 comprises VSV-g, pMD Gag/Pol, RSV-REV; and the centrifugation is performed using Beckman ultracentrifuge and SW28 head.

8. The method of preparing said universal CAR-T cell according to claim 5 or 6, wherein said human PBMC is mononuclear cells derived from cord blood or adult peripheral blood.

9. A formulation comprising said universal CAR-T cell according to claim 1 or claim 2.

10. Use of said universal CAR-T cell according to any one of claim 1 or claim 2 in the preparation of a medicament for treating or preventing tumor.
